# EUROPEAN PATENT APPLICATION

(11) **EP 4 414 450 A1**
(43) Date of publication of application: **14.08.2024**
(21) Application number: 22878485.6
(22) Date of filing: 03.10.2022
(51) Int. Cl.: C12N 9/88, C12N 9/10

(54) **LIQUID TRANSGLUTAMINASE ENZYME PREPARATION**

(30) Priority: 04.10.2021 JP 2021163371
(71) Applicant: Amano Enzyme Inc., Nagoya-shi Aichi 460-8630 (JP)
(72) Inventor: SAKAI, Kiyota, Kakamigahara-shi, Gifu 509-0109 (JP)
(74) Representative: Müller-Boré & Partner Patentanwälte PartG mbB
(86) International application number: PCT/JP2022/037019
(87) International publication number: WO 2023/058616

(57) **Abstract**

The purpose of the present invention is to provide a new means for stabilizing a transglutaminase. The present invention stabilizes a transglutaminase by causing the transglutaminase to coexist with sodium carbonate and/or sodium sulfate under a condition of a pH of 6.5 or less.

## Description

### TECHNICAL FIELD

The present invention relates to a liquid transglutaminase enzyme preparation and a production method of a crosslinked protein. More specifically, the present invention relates to a liquid transglutaminase enzyme preparation having improved thermal stability, and a production method of a crosslinked protein in a reaction system having improved thermal stability of a transglutaminase.

### BACKGROUND ART

The transglutaminase is an acyltransferase to which EC 2.3.2.13 is assigned as an EC number, and crosslinks a protein by catalyzing an acyl transfer reaction between the γ-carboxamide group of a glutamine residue and the ε-amino group of a lysine residue in the protein. Due to this property, the transglutaminase is an enzyme with high utility in modifying food proteins and the like.

It is desirable that an enzyme preparation containing a transglutaminase as an active ingredient can be used with high enzyme utilization efficiency in modifying food proteins and the like by being formulated in a highly stable formulation.

For example, Patent Document 1 describes a liquid enzyme formulation in which a milk protein crosslinking and/or modifying enzyme such as a transglutaminase is stabilized by being contained in a polyol-water suspension containing 25 to 100%

(w/w) polyol and having a pH value within the range of 4.4 to 5.1; and Patent Document 2 describes a liquid enzyme preparation in which a transglutaminase is stabilized by being contained together with 5 wt% or more of one or two or more members selected from the group consisting of glycine, proline, serine, a glutamic acid salt, an aspartic acid salt, and an organic acid salt.

### PRIOR ART DOCUMENTS

### PATENT DOCUMENTS

Patent Document 1: International Publication No. WO 2013/064736
Patent Document 2: International Publication No. WO 2019/182123

### SUMMARY OF THE INVENTION

### PROBLEMS TO BE SOLVED BY THE INVENTION

In liquid enzyme preparations of a transglutaminase reported so far, the formulation restriction is strict, or the use of the enzyme preparation is limited only for food because an organic compound such as an amino acid or an organic acid is used as a stabilizer, and thus a new stabilizing means has been desired.

Therefore, an object of the present invention is to provide a new means for stabilizing a transglutaminase.

### MEANS FOR SOLVING THE PROBLEM

As a result of intensive studies to solve the above problems, the present inventors have found that a transglutaminase exhibits excellent stability in the presence of sodium carbonate and/or sodium sulfate and under a condition that pH is 6.5 or less. The present invention has been completed by further conducting studies based on this finding. That is, the present invention provides inventions of the following aspects.

Item 1. A liquid transglutaminase enzyme preparation including (a) a transglutaminase, and (b) sodium carbonate and/or sodium sulfate, in which pH is 6.5 or less at 25°C.

Item 2. The enzyme preparation according to Item 1, in which the component (b) is contained in an amount of 0.001 to 1 mmol with respect to 1 U of the component (a).

Item 3. The enzyme preparation according to Item 1 or 2, being used as a protein crosslinking agent.

Item 4. A production method of a crosslinked protein, including a step of allowing (a) a transglutaminase to act on a protein in a presence of (b) sodium carbonate and/or sodium sulfate and under a condition that pH is 6.5 or less at 25°C.

Item 5. The production method according to Item 4, in which the component (b) is used in an amount of 0.001 to 1 mmol with respect to 1 U of the component (a).

Item 6. The production method according to Item 4 or 5, in which the step is performed at a temperature of 45°C or higher.

### BRIEF DESCRIPTION OF THE DRAWING

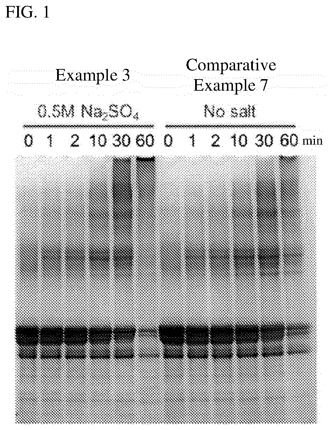

Fig. 1 is a result showing that sodium sulfate enhances the protein crosslinking effect of a transglutaminase in the production method of a crosslinked protein of the present invention.

### EMBODIMENTS OF THE INVENTION

### 1. Liquid transglutaminase enzyme preparation

The liquid transglutaminase enzyme preparation of the present invention is characterized by containing (a) a transglutaminase (hereinafter, also described as "component (a)") and (b) sodium carbonate and/or sodium sulfate (hereinafter, also described as "component (b)"), and having a pH of 6.5 or less at 25°C. Hereinafter, the liquid transglutaminase enzyme preparation of the present invention will be described in detail.

### (a) Transglutaminase

The liquid transglutaminase enzyme preparation of the present invention contains a transglutaminase as the component (a). The transglutaminase is an enzyme having an activity of crosslinking a protein by catalyzing an acyl transfer reaction between the γ-carboxamide group of a glutamine residue and the ε-amino group of a lysine residue in a protein.

Specific examples of the transglutaminase used in the present invention are not particularly limited. Specific examples of the transglutaminase include a microorganism-derived transglutaminase, specifically, a transglutaminase derived from the genus Streptomyces such as Streptomyces ladakanum, Streptomyces cinnamoneus, Streptomyces griseocarneus, Streptomyces lavendulae, or Streptomyces lydicus (See, for example, JP S64-27471 A.), a transglutaminase derived from Kutzneria such as Kutzneria albida (See, for example, JP 2020-195397 A.), and a transglutaminase derived from the genus Longimycelium such as Longimycelium tulufanense; a mammal-derived transglutaminase (See, for example, JP H01-50382 B.); a fish-derived transglutaminase (See, for example, JP H06-113844 A.); a recombinant transglutaminase obtained using recombinant DNA technology (See, for example, JP H05-199883 A and JP 2004-97099 A.); and transglutaminase having such a structure that a pro-sequence peptide of transglutaminase is bound to matured transglutaminase (See, for example, WO 2009/101762 A1). These transglutaminases may be used alone, or may be used in combination of two or more thereof.

Among these transglutaminases, from the viewpoint of further enhancing the stabilization effect, a microorganism-derived transglutaminase is preferable, and a transglutaminase derived from the genus Streptomyces is more preferable, and a transglutaminase derived from the species Streptomyces mobaraensis is still more preferable.

The content (total amount) of the component (a) in the liquid transglutaminase enzyme preparation is not particularly limited as long as the effect of the component (a) can be exhibited in use of the enzyme preparation, and is, for example, 1 to 100,000 U, preferably 10 to 50,000 U, more preferably 100 to 10,000 U, still more preferably 250 to 5,000 U, and still more preferably 500 to 1,500 U in 100 ml of the liquid transglutaminase enzyme preparation.

For the activity value of the transglutaminase, 1 unit (U) is defined as the enzyme activity of producing 1 µmol of hydroxamic acid for 1 minute when benzyloxycarbonyl-L-glutaminylglycine and hydroxylamine are used as a substrate.

### (b) Sodium carbonate and/or sodium sulfate

The liquid transglutaminase enzyme preparation of the present invention contains sodium carbonate and/or sodium sulfate as the component (b). The component (b) enhances the stability of the transglutaminase in the liquid transglutaminase enzyme preparation whose pH is adjusted to 6.5 or less.

Among sodium carbonate and sodium sulfate, sodium sulfate is preferable from the viewpoint of further enhancing the stabilization effect.

The content (total amount) of the component (b) in the liquid transglutaminase enzyme preparation is not particularly limited as long as the stabilization effect of the transglutaminase is obtained, and may be appropriately set depending on how much the stabilization effect is required.

The content (total amount) of the component (b) in the liquid transglutaminase enzyme preparation is, as a specific example, 0.001 to 1 mmol with respect to 1 U of the component (a), and is preferably 0.005 to 0.5 mmol, more preferably 0.01 to 0.1 mmol, and further preferably 0.03 to 0.07 mmol, from the viewpoint of further enhancing the stabilization effect.

The content (total amount) of the component (b) in the liquid transglutaminase enzyme preparation is, as another specific example, 0.01 to 10 M, and is preferably 0.05 to 5 M, more preferably 0.1 to 1 M, and still more preferably 0.3 to 0.7 M, from the viewpoint of further enhancing the stabilization effect.

### Other components

The liquid transglutaminase enzyme preparation of the present invention may further contain, as appropriate, at least one of a base material and an additive, specifically, one or more of the following components, depending on the preparation form and/or use.

The liquid transglutaminase enzyme preparation of the present invention typically contains water as the base material. The water is not particularly limited, and examples thereof include purified water, deionized water, and tap water.

Examples of the additive include a stabilizer, a buffer, a pH adjuster, a preservative, and an antiseptic.

As the stabilizer, propylene glycol, ascorbic acid, other known stabilizers for a transglutaminase (specifically, one or two or more selected from the group consisting of glycine, proline, serine, a glutamic acid salt, an aspartic acid salt, and an organic acid salt (more specifically, those blended in an amount of 5 wt% or more in the total amount thereof), and a polyol (more specifically, those blended in an amount of 25 wt% or more)), and the like are allowed to be blended. In the present invention, since the stabilization effect of the transglutaminase is excellent due to the coexisting component (b) under a predetermined pH condition, these stabilizers are not contained in a preferred embodiment.

The buffer is not particularly limited as long as the pH of the liquid transglutaminase enzyme preparation can be adjusted to 6.5 or less alone or in combination with a pH adjuster, and examples thereof include a phosphate buffer, a citrate buffer, a phthalate buffer, a maleate buffer, a glycine-HCl buffer, a carbonate buffer, and a Good buffer, and preferably include a Good buffer. Examples of the Good buffer include N-(2-acetamide)-2-aminoethanesulfonic acid (ACES), N,N-bis(2-hydroxyethyl)-2-aminoethanesulfonic acid (BES), N-cyclohexyl-2-aminoethanesulfonic acid (CHES), N-cyclohexyl-3-aminopropanesulfonic acid (CAPS), 2-[4-(2-hydroxyethyl)-1-piperazinyl]ethanesulfonic acid (HEPES), 2-morpholinoethanesulfonic acid (MES), piperazine-1,4-bis(2-ethanesulfonic acid) (PIPES), N-tris(hydroxymethyl)methyl-2-aminomethanesulfonic acid (TES), N-cyclohexyl-3-aminopropanesulfonic acid (CAPS), N-cyclohexyl-2-hydroxy-3-aminopropanesulfonic acid (CAPSO), 3-[N,N-bis(2-hydroxyethyl)amino]-2-hydroxypropanesulfonic acid (DIPSO), 3-[4-(2-hydroxyethyl)-1-piperazinyl]propanesulfonic acid (EPPS), 2-hydroxy-3-[4-(2-hydroxyethyl)-1-piperazinyl]propanesulfonic acid (HEPPSO), 3-morpholinopropanesulfonic acid (MOPS), 2-hydroxy-3-morpholinopropanesulfonic acid (MOPSO), piperazine-1,4-bis(2-hydroxy-3-propanesulfonic acid) (POPSO), N-tris(hydroxymethyl)methyl-3-aminopropanesulfonic acid (TAPSO), N-tris(hydroxymethyl)methyl-3-aminopropanesulfonic acid (TAPS), N-(2-acetamide)iminodiacetic acid (ADA), N,N-bis(2-hydroxyethyl)glycine (Bicine), N-[tris(hydroxymethyl)methyl]glycine (Tricine), and MES is preferable.

The pH adjuster is not particularly limited as long as it can adjust the pH of the liquid transglutaminase enzyme preparation to 6.5 or less, and examples thereof include acids such as hydrochloric acid, phosphoric acid, acetic acid, and sulfuric acid, and bases such as sodium hydroxide, potassium hydroxide, sodium carbonate, sodium hydrogen carbonate, and aqueous ammonia.

Examples of the preservative include phenol, benzalkonium chloride, benzyl alcohol, chlorobutanol, and methylparaben. Examples of the antiseptic include benzoate (alkali metal salts such as potassium salt and sodium salt), sorbate (alkali metal salts such as potassium salt and sodium salt), ethanol, benzalkonium chloride, paraoxybenzoic acid, and chlorobutanol.

### pH

The liquid transglutaminase enzyme preparation of the present invention has a pH of 6.5 or less at 25°C. From the viewpoint of further enhancing the stabilization effect, the pH is preferably 6.3 or less, more preferably 6.2 or less, and still more preferably 6.1 or less. The lower limit of the pH range is, for example, 4.0 or more, and is preferably 4.8 or more, more preferably 5.3 or more, still more preferably 5.8 or more, from the viewpoint of further enhancing the stabilization effect.

### Application

The liquid transglutaminase enzyme preparation of the present invention is used for any use for obtaining the effect of a transglutaminase. Preferably, the liquid transglutaminase enzyme preparation of the present invention is used as a protein crosslinking agent, and in a more specific example, is used as a protein material modifier for obtaining a property change of a protein material based on protein crosslinking. Preferred examples of the method for use as a protein-crosslinking agent include the following "2. Production method of crosslinked protein".

### 2. Production method of crosslinked protein

As described above, the transglutaminase is stabilized by coexisting with sodium carbonate and/or sodium sulfate under the condition of pH 6.5 or less. Therefore, when a protein-crosslinking reaction is performed using the transglutaminase, the reaction system is set to a condition of pH 6.5 or less, and sodium carbonate and/or sodium sulfate is contained in the reaction system, thereby excellent transglutaminase utilization efficiency can be obtained.

Accordingly, the present invention also provides a production method of a crosslinked protein, including a step of allowing (a) a transglutaminase to act on a protein in a presence of (b) sodium carbonate and/or sodium sulfate and under a condition that pH is 6.5 or less at 25°C.

In the step, specifically, a reaction mixture containing a protein, the component (a), the component (b), and water and having the pH adjusted to 6.5 or less at 25°C is subjected to a reaction condition under which the transglutaminase advances a crosslinking reaction of the protein.

There is no particular limitation on the origin, properties, and the like of the protein as a substrate as long as the protein can undergo the crosslinking action by the transglutaminase. Specific examples of the protein include: natural proteins such as animal proteins, proteins derived from insects, plant proteins, proteins derived from fungi, and proteins derived from algae; and artificial proteins.

The animal protein is not particularly limited, and examples thereof include proteins derived from vertebrates. Proteins derived from vertebrates are not particularly limited, but preferable examples thereof include proteins derived from mammals, and more specifically, milk proteins such as casein and β-lactoglobulin; egg proteins such as ovalbumin; meat proteins such as myosin and actin; blood proteins such as serum albumin; and tendon proteins such as gelatin and collagen.

The protein derived from insects is not particularly limited, and examples thereof include proteins derived from crickets and proteins derived from silkworms.

Examples of the plant protein include proteins derived from legume crops such as soy beans, green peas, lentils, chickpeas, black beans, and fava beans, and proteins derived from cereal crops such as wheat, barley, oat, rye, and rice; proteins derived from nuts and seeds such as almond, peanut, cannabis seed (hemp seed: so-called industrial hemp, and specifically refers to a variety that does not contain THC (tetrahydrocannabinol), which causes perceptual modification or has a low THC concentration. Since an industrial hemp does not contain THC or have a low concentration thereof, an industrial hemp has no efficacy as a perceptual transformant and cannot lead to abuse.), chia seed, quinoa, and amaranthus.

Examples of the protein derived from fungi include proteins derived from yeast, proteins derived from filamentous fungi, and proteins derived from true fungi (mycoproteins derived from mushrooms, etc.).

Examples of the artificial protein include proteins synthesized chemically, enzymatically, or genetically.

In the present invention, the protein as a substrate for the crosslinking reaction may be in a purified state or in the state of being contained in a known protein material to which a transglutaminase is generally applied. Therefore, the reaction mixture may contain the protein material itself serving as a protein source. Examples of the protein material include food and drink materials (including feed materials), fiber materials, chemical materials (photographic films, tanning, and the like), perfumery materials, and pharmaceutical materials.

The details of the component (a) contained in the reaction mixture and the amount of the component (b) used with respect to the amount of the component (a) used are as described above as the details of the component (a) and the content of the component (b) per 1 U of the component (a) in the "1. Liquid transglutaminase enzyme preparation", respectively.

The reaction mixture has a pH of 6.5 or less at 25°C. From the viewpoint of further enhancing the stabilization effect of the transglutaminase, the pH is preferably 6.3 or less, more preferably 6.2 or less, and still more preferably 6.1 or less. The lower limit of the pH range is, for example, 4.0 or more, and is preferably 4.8 or more, more preferably 5.3 or more, still more preferably 5.8 or more, from the viewpoint of further enhancing the stabilization effect of the transglutaminase.

The reaction mixture may be prepared after a protein (or a protein material containing the protein), the component (a), the component (b), and as necessary, an appropriate solvent (typically, water) and at least one of a buffer and/or a pH adjuster for adjusting the pH of the reaction mixture to 6.5 or less are prepared, respectively; or may be prepared after a protein (or a protein material containing the protein), the enzyme preparation described in the " 1. Liquid transglutaminase enzyme preparation", and as necessary, an appropriate solvent (typically, water) and at least one of a buffer and/or a pH adjuster for adjusting the pH of the reaction mixture to 6.5 or less are prepared, respectively.

The reaction mixture is only required to be constructed such that the protein and the component (a) are in contact with each other under a condition where pH is 6.5 or less at 25°C and in a state of coexisting with the component (b), and may be homogeneous or heterogeneous.

The temperature condition under which the reaction mixture is provided is not particularly limited, and may be appropriately determined according to the optimal temperature of the component (a) and the like, but in the production method of the present invention, the heating condition is preferable because the thermal stability of the transglutaminase can be favorably maintained during the crosslinking reaction. Specific temperature conditions include, for example, 45°C or higher, and preferably 48°C or higher. The upper limit of the temperature condition is not particularly limited, and is, for example, 65°C or lower, preferably 60°C or lower, more preferably 55°C or lower, and still more preferably 52°C or lower.

The time for performing the above step (working time) is not particularly limited, and is, for example, 10 to 600 minutes, 20 to 300 minutes, 30 to 180 minutes, preferably 40 to 120 minutes, more preferably 50 to 70 minutes.

### EXAMPLES

Hereinafter, the present invention will be described with reference to Examples, but the present invention is not limited to these Examples.

### <Reagents and materials>

**[Table 1]**

| Protein | Milk casein (Casein, Bovine Milk, Carbohydrate and Fatty Acid Free) (Merck & Co., Inc.) |
|---|---|
| Transglutaminase | Streptomyces mobaraensis-derived transglutaminase |
| Sodium carbonate | Sodium carbonate, Guaranteed Reagent (FUJIFILM Wako Pure Chemical Corporation) |
| Sodium sulfate | Sodium sulfate, Guaranteed Reagent (FUJIFILM Wako Pure Chemical Corporation) |
| Buffer solution | 200 mM MES buffer solution |

### <Method for measuring transglutaminase activity>

The enzyme activity of transglutaminase was measured by the method described below using benzyloxycarbonyl-L-glutaminylglycine and hydroxylamine as the substrate.

A substrate solution was prepared by dissolving 2.42 g of 2-amino-2-hydroxymethyl-1.3-propanediol, 0.70 g of hydroxyammonium hydrochloride, 0.31 g of reduced glutathione, and 1.01 g of Z-Gln-Gly (benzyloxycarbonyl-L-glutaminylglycine) in distilled water, and setting the total amount thereof to 100 mL (pH 6.0).

A coloring solution was prepared by mixing 30 mL of a 3 M hydrochloric acid solution, 30 mL of a 12 wt% trichloroacetic acid solution, and 30 mL of a 5 wt% iron (III) chloride solution.

A sample solution was prepared by diluting the enzyme with a 200 mM MES buffer (pH 6.0) into an appropriate concentration (specifically, the concentration at which the concentration of the salt is about 1 mM).

The reaction was performed at 37°C for 10 minutes after adding and mixing 100 µL of the substrate solution into 10 µL of the sample solution. The reaction was stopped by adding 100 µL of the coloring solution and an Fe complex was formed, and then the absorbance at 525 nm was measured. As a control, the previously heatinactivated sample solution was reacted in the same manner and measured for absorbance, thereby determining the absorbance difference from the non-inactivated sample solution. Separately, a calibration curve was prepared using L-glutamic acid-γ-monohydroxamic acid instead of the sample solution, and the amount of generated hydroxamic acid was determined from the absorbance difference. The enzyme activity to produce 1 µmol hydroxamic acid for 1 minute was defined as 1 unit (1 U).

### <Test Example 1>

### (1) Preparation of liquid transglutaminase enzyme preparation

Liquid transglutaminase enzyme preparations having the formulation shown in Table 2 were prepared. In the preparation, a 200 mM MES buffer solution was used, and the pH was adjusted using NaOH such that the pH (final pH) was 6.0 at 25°C. Therefore, the liquid transglutaminase enzyme preparations having the formulation shown in Table 2 contain water as a solvent, MES as a buffer, and NaOH as a pH adjuster, in addition to the components shown in Table 2.

### (2) Thermal stability test

The prepared liquid transglutaminase enzyme preparation was stored at 50°C for 80 minutes. Thereafter, the preparation was ice-cooled, and measured for transglutaminase activity. The transglutaminase activity residual ratio (%) was calculated after storage, the transglutaminase activity before storage being 100%. The higher the activity residual ratio is, the higher the thermal stability can be evaluated.

### (3) Results

Table 2 shows the results. As shown in Table 2, the effect of improving thermal stability was confirmed when sodium carbonate and sodium sulfate were used.

**[Table 2]**

| | | | Comparative Example | Example | | Comparative Example | | | | |
|---|---|---|---|---|---|---|---|---|---|---|
| | | | 1 | 1 | 2 | 2 | 3 | 4 | 5 | 6 |
| (a) | | Transglutaminase | 1000 U/100 ml | | | | | | | |
| | (b) | Sodium carbonate | - | 0.5 M | - | - | - | - | - | - |
| | | Sodium sulfate | - | - | 0.5 M | - | - | - | - | - |
| Salt | | Sodium chloride | - | - | - | 0.5 M | - | - | - | - |
| | | Sodium bromide | - | - | - | - | 0.5 M | - | - | - |
| | | Sodium nitrate | - | - | - | - | - | 0.5 M | - | - |
| | | Sodium iodide | - | - | - | - | - | - | 0.5 M | - |
| | | Sodium thiocyanate | - | - | - | - | - | - | - | 0.5 M |
| (a) Amount of salt per 1 U (mmol/U) | | | 0 | 0.05 | 0.05 | 0.05 | 0.05 | 0.05 | 0.05 | 0.05 |
| Activity residual ratio | | | 40% | 46% | 55% | 38% | 25% | 11% | 0% | 0% |

### <Test Example 2>

Sodium sulfate was dissolved in a 200 mM MES buffer solution and the pH was adjusted to 6.0 at 25°C. In this solution, milk casein was dissolved and the pH was adjusted to 6.0 at 25°C. Then the transglutaminase was added, thereby preparing a reaction mixture containing milk casein (5 wt%), the transglutaminase (1000 U/100 ml), and sodium sulfate (0.5 M: 0.05 mmol with respect to 1 U of the transglutaminase) and having a pH of 6.0 at 25°C.

The reaction mixture was subjected to a condition of 50°C and 60 minutes to perform a reaction step of crosslinking the protein (Example 3). On the other hand, the same procedure as in Example 3 was carried out except that sodium sulfate was not used (Comparative Example 7). Before the reaction and 1, 2, 10, 30, and 60 minutes after the start of the reaction, the reaction mixture was sampled, mixed with an SDS-PAGE sample buffer, boiled, and then ice-cooled. The sample thus obtained was subjected to SDS-PAGE to evaluate the crosslinking activity.

The results are shown in Fig. 1. In the electropherogram, the closer the position of the band is to the origin, the more the protein crosslinking reaction proceeds. As shown in Fig. 1, when sodium sulfate was allowed to coexist under the condition of pH 6.0 (Example 3), the protein crosslinking effect by the transglutaminase was remarkably improved.

## Claims

1. A liquid transglutaminase enzyme preparation comprising (a) a transglutaminase, and (b) sodium carbonate and/or sodium sulfate, wherein pH is 6.5 or less at 25°C.

2. The enzyme preparation according to claim 1, wherein the component (b) is contained in an amount of 0.001 to 1 mmol with respect to 1 U of the component (a).

3. The enzyme preparation according to claim 1, being used as a protein crosslinking agent.

4. A production method of a crosslinked protein, comprising a step of allowing (a) a transglutaminase to act on a protein in a presence of (b) sodium carbonate and/or sodium sulfate and under a condition that pH is 6.5 or less at 25°C.

5. The production method according to claim 4, wherein the component (b) is used in an amount of 0.001 to 1 mmol with respect to 1 U of the component (a).

6. The production method according to claim 4, wherein the step is performed at a temperature of 45°C or higher.
